# EUROPEAN PATENT SPECIFICATION

(11) **EP 1 627 658 B1**
(45) Date of publication and mention of the grant of the patent: **21.03.2012**
(21) Application number: 04425633.7
(22) Date of filing: 16.08.2004
(51) Int. Cl.: A61M 39/22

(54) **Two-way T-like valve for an infusion line**
T-formiges Zweiwegventil für eine Infusionsleitung
Vanne à 2 voies en forme de "T" pour une ligne d'infusion

(43) Date of publication of application: 22.02.2006
(73) Proprietor: Fresenius Kabi Deutschland GmbH, 61352 Bad Homburg v.v. Höhe (DE)
(72) Inventor: Reiter, Reinhold, 26013 Crema (Cremona) (IT); Vaiano, Andrea, 23807 Merate (Lecco) (IT); Fini, Massimo, 41037 Mirandola (Modena) (IT)
(74) Representative: Rondano, Davide

(56) References cited:
- EP-A- 1 129 682
- EP-A- 1 221 322
- WO-A-92/21403
- WO-A-03/029706
- US-A- 4 169 491

## Description

The present invention relates to a two-way T-like valve for an infusion line, of the type in which a rotational movement is used to open or close a port.

Infusion lines are commonly used in medical therapy to supply the patient with the necessary nutrients and medicaments. An infusion line typically includes:
- a bag or bottle containing a liquid solution with the nutrients or medicaments to be fed,
- an injection site for connection to the blood stream of the patient, the bag or bottle being positioned at a higher level than the injection site so that the solution is delivered by gravity,
- a circuit (generally a plastics flexible tube) connecting the bag or bottle to the injection site, and
- a roller clamp fitted on the flexible tube for adjusting the feeding rate of the solution.

During infusion it may be necessary to inject medicaments into the main infusion line. In order to avoid possible interactions between the active ingredients of the medicaments and the infusion fluid it is usually necessary to interrupt the infusion process and flush the infusion line, which is time consuming and costly.

WO 92/21403 discloses a T-like valve for an infusion line, where rotational movement is used to open a side port for putting it into fluid connection with a main line or alternatively close the side port for preventing fluid connection between the side port and the main line. This known valve, however, suffers from the disadvantage that it does not allow simultaneously to open the side port and close the main line. In fact, the valve only provides for a first position in which both the side port and the main line are open, so that fluid connection is established therebetween, and a second position in which the side port is closed and the main line is open.

A valve for an infusion line according to the preamble of independent Claim 1 is known from WO 03/029706. More specifically, WO 03/029706 discloses a four-port four-way valve comprising a housing and a body, wherein the housing has a central axial cavity and three connectors extending radially from the housing and in fluid communication with the cavity and wherein the body is rotatably accommodated in the central axial cavity of the housing about the axis of the cavity. The body has an axial port and three radial ports, namely a first and a second port radially aligned with one another and a third port perpendicular to the other two, as well as two independent fluid passages that can carry fluid between two different pairs of ports, simultaneously. A first passage extends between the axial port and the third radial port, while the second passage extends between the first and second radial ports. The valve body may be selectively rotated such that the third radial port, and consequently the axial port, is put in fluid communication with any one of the three connectors.

It is therefore the object of the present invention to provide a T-like valve for an infusion line which can overcome the disadvantage of the prior art discussed above. A further object of the invention is to provide an injection site valve which has a simple structure and can be manufactured at a lower cost than the prior art.

These and other objects are fully achieved according to the invention by a T-like valve having the characteristics set forth in Claim 1. Further advantageous characteristics are specified in the dependent claims.

The characteristics and the advantages of the invention will appear from the detailed description which follows, given purely by way of non-limiting example with reference to the appended drawings, in which:
- Figure 1 is a perspective view of a T-like valve for an infusion line according to a preferred embodiment of the present invention, the valve being arranged in a first position in which a side port is closed and a main line is open;
- Figure 2 is an axially sectioned, perspective view of the valve of Figure 1;
- Figure 3 is an axially sectioned, elevation view of the valve of Figure 1;
- Figure 4 is a transversely sectioned, elevation view of the valve of Figure 1;
- Figure 5 is a perspective view of the valve of Figure 1, showing a second position of the valve in which the side port is open and the main line is closed;
- Figure 6 is an axially sectioned, perspective view of the valve of Figure 5; and
- Figure 7 is an axially sectioned, elevation view of the valve of Figure 5.

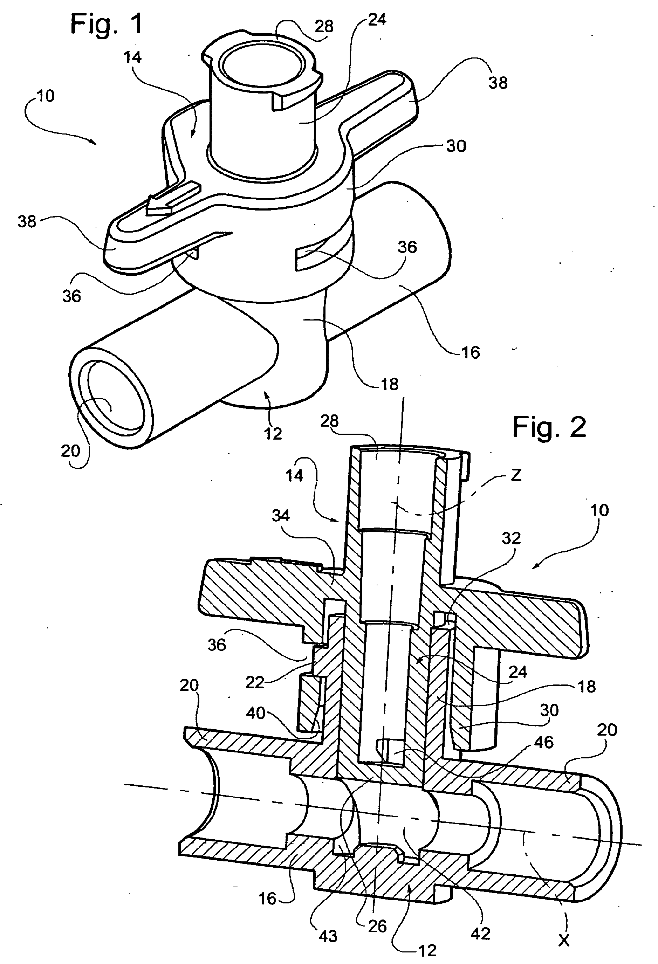

With reference to Figures 1 to 7, a two-way T-like valve for an infusion line according to a preferred embodiment of the present invention is generally indicated 10 and basically includes a coupling housing 12 and a valve body 14.

The coupling housing 12 has an inverted T-shape configuration, with a first tubular segment 16 of longitudinal axis X and a second tubular segment 18 of longitudinal axis Z arranged perpendicular to the axis X of the first segment and debouching into a middle portion of this segment. The first segment 16 forms at its opposite ends two connecting portions 20 for connection with a main line (not shown). The second segment 18 is open at both its ends and is provided on its outer cylindrical surface with a pin-like projection 22 for securing axially the valve body 14 to the housing 12 and limiting the rotation of the valve body 14 relative to the housing 12, as will be explained in greater detail later on.

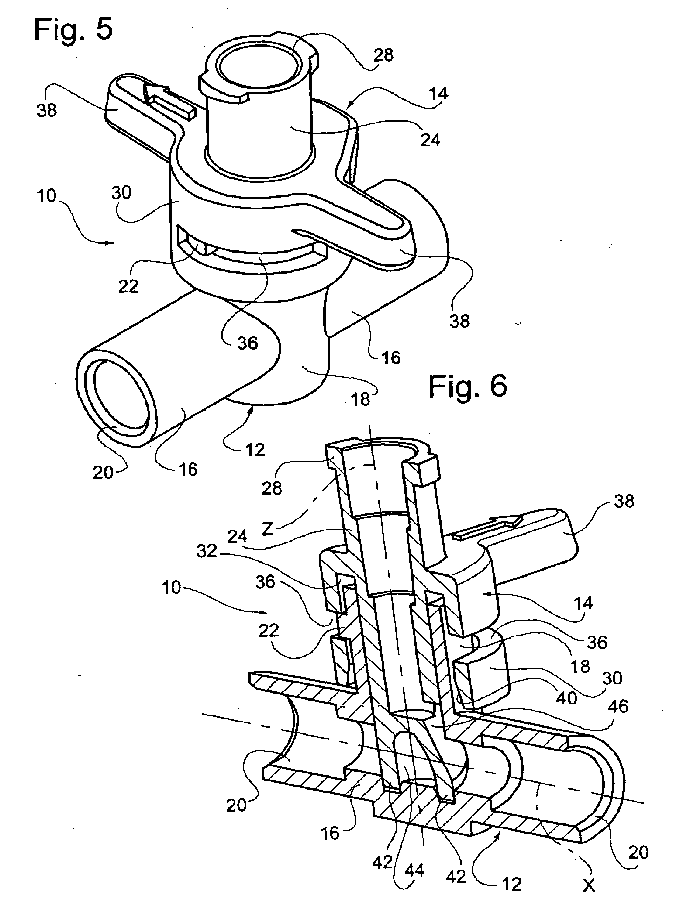

The valve body 14 comprises a first, inner tubular segment 24, partially closed at its lower end by a bottom wall 26 and forming at its upper end a connecting portion 28 for connection with a side or infusion line (not shown), through which drugs or medicaments are to be supplied. A lower portion of the first, inner tubular segment 24 is rotatably mounted in the second segment 18 of the housing 12 around the longitudinal axis Z of this segment. The valve body 14 further comprises a second, outer tubular segment 30 arranged coaxial with the first and radially spaced therefrom so as to define a seat 32 for receiving the second segment 18 of the housing 12. The outer tubular segment 30 is connected to the inner one by a top wall 34. A horizontal groove 36 is provided in the circular cylindrical wall of the outer tubular segment 30, which groove extends approximately through a 90-degree angle. The pin-like projection 22 of the second segment 18 of the housing 12 engages by snap-fitting into the groove 36 in such a manner that the valve body 14 is axially locked onto the housing 12 but can rotate relative thereto through a 90-degree angle, between a first position (illustrated in Figures 1 to 4) and a second position (illustrated in Figures 5 to 7) perpendicular to the first. The engagement of the pin-like projection 22 in the groove 36 thus ensures that the valve body 14 is guided and limited in its rotational movement relative to the housing 12.

A pair of gripping arms 38 extend radially in opposite directions from the outer tubular segment 30 for helping the user in rotating the valve body 14 between the above-defined first and second position. The bottom end of the circular cylindrical wall of the outer tubular segment 30 has a tapering lead-in inner surface 40 for making it easier to connect the valve body 14 with the housing 12 by snap-fitting the projection 22 into the groove 36.

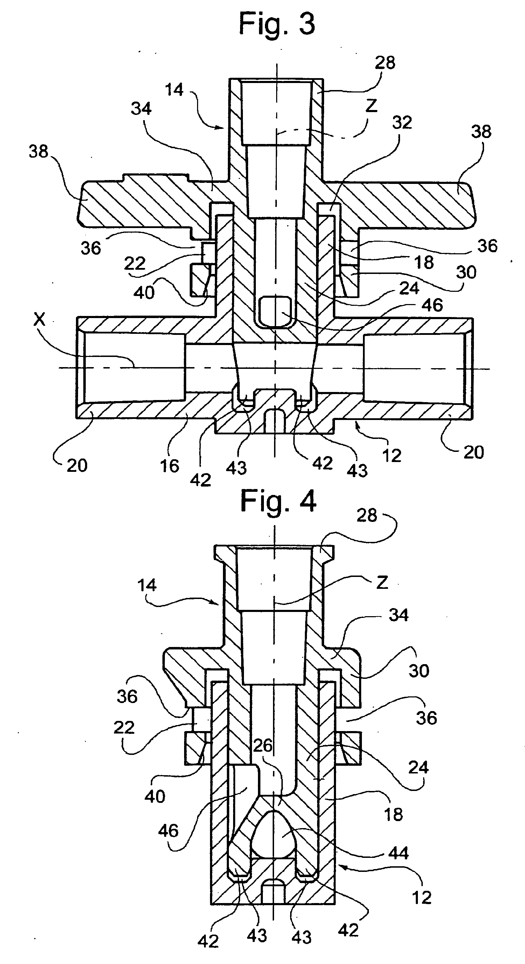

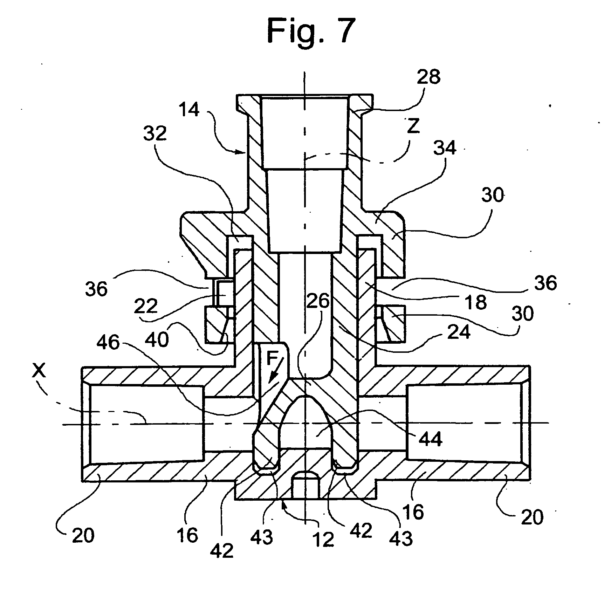

As can be seen better in Figures 4 and 6, the inner tubular segment 24 of the valve body 14 forms a pair of prong-like protrusions 42 which extend downwards from the bottom wall 26 so as to form a fork-like portion. The free ends of the protrusions 42 engage in a seat 43 formed as a circumferential groove in a middle portion of the first tubular segment 16 of the housing 12 so as to ensure fluid-tightness when the valve is in the second position (see for example Figure 7).

The shape and size of the prong-like protrusions 42 are such that they are able to close the first tubular segment 16 of the coupling housing 12 when the valve body 14 is arranged in the above-defined second position. A U-like cavity or channel 44 defined between the two prong-like protrusions 42 allows fluid flowing in the main line to pass through the first tubular segment 16 when the valve body 14 is arranged in the above-defined first position. Therefore, the groove 36 has to be shaped in such a manner that the pin-like projection 22 of the housing 12 abuts against a first end of the groove 36 when the channel 44 is arranged parallel to the longitudinal axis X of the first segment 16 (first position) for opening the main line, whereas the pin-like projection 22 abuts against the opposite end of the groove 36 when the channel 44 is arranged perpendicular to the longitudinal axis X (second position) for closing the upstream side of the main line. Preferably, the gripping arms 38 of the valve body 14 extend parallel to the channel 44, so that in the first position of the valve they are arranged parallel to the longitudinal axis X of the first segment 16 (thus indicating that the main line is open), whereas in the second position of the valve they are arranged perpendicular to the longitudinal axis X of the first segment 16 (thus indicating that the main line is closed).

In order to allow fluid connection between the infusion line and the main line when the latter is closed by one of the prong-like protrusions 42 of the inner tubular portion 24 of the valve body 14 (second position of the valve), a radial through hole 46 is provided which is arranged on the same side of the tubular portion 24 as one of the protrusions 42 and leads into the first tubular segment 16 of the housing 12, as shown in Figures 6 and 7. Accordingly, fluid entering into the inner tubular portion 24 of the valve body 14 from the infusion line is allowed to pass through the hole 46 in a substantially radial direction (Figure 7, arrow F) with respect to the axis of the channel 44.

The operation of the valve will be described now in brief. When the valve 10 is arranged in the first position (Figures 1 to 4), fluid is free to flow through the main line, since the channel 44 of the valve body 14 is aligned with the first tubular segment 16 of the coupling housing 12. On the other hand, infusion of drugs or medicaments into the main line from the infusion line is prevented, since the hole 46 in the inner tubular portion 24 of the valve body 14 is not turned towards the first tubular segment 16 of the housing 12.

Upon 90-degree rotation of the valve body 14, the valve 10 reaches the above-defined second position (Figures 5 to 7), in which the channel 14 is oriented perpendicular to the first tubular segment 16 of the coupling housing 12, the upstream prong-like protrusion 42 (the right-hand one in Figure 7) closes the flow area of an upstream portion of the first tubular segment 16, and the through hole 46 is open to a downstream portion of the first tubular segment 16 (the left-hand one in Figure 7). Therefore, fluid flow through the main line is prevented, whereas infusion of drugs or medicaments from the infusion line into the main line is allowed through the inner tubular segment 24 of the valve body 14 and the hole 46.

Naturally, the principle of the invention remaining unchanged, embodiments and manufacturing details may be varied widely with respect to those described and illustrated purely by way of non-limiting example.

## Claims

1. A two-way T-like valve (10) for an infusion line, including
- a coupling housing (12) having an inverted T-shape configuration, with
a first tubular segment (16) extending along a first axis (X) and forming at its opposite ends first and second connecting portions (20) for connection with a first, main line, and
a second tubular segment (18) extending along a second axis (Z) perpendicular to the first axis (X) and debouching into a middle portion of the first segment (16);
- a valve body (14) rotatably mounted in the second segment (18) of the housing (12) around the second axis (Z) between a first and a second position; and
- a third connecting portion (28) for connection with a second, infusion line; wherein the valve body (14) has an end portion (42) which is contained within the first tubular segment (16) of the housing (12) and defines a channel (44) arranged substantially transversely with respect to the second axis (Z), in such a manner that when the valve body (14) is in the said first position the channel (44) is substantially aligned with the first axis (X) and when the valve body (14) is in the said second position the channel (44) is arranged substantially perpendicular to the first axis (X); and
wherein the valve body (14) has a radial through hole (46) configured so as to connect the third connecting portion (28) with a downstream portion of the first segment (16) of the housing (12) when the valve body (14) is in the second position, whereby the upstream side of the main line is closed and fluid flow is allowed from the infusion line to the downstream side of the main line;
the valve being **characterized in that** said end portion (42) of the valve body (14) is a fork-like portion having a pair of prong-like protrusions (42) between which the channel (44) is defined, and **in that** the free ends of the prong-like protrusions (42) of the valve body (14) engage in a seat (43) formed as a circumferential groove in a middle portion of the first tubular segment (16) of the housing (12) so as to ensure fluid-tightness when the valve is in the said second position.

2. A valve according to Claim 1, wherein the radial through hole (46) is arranged on the same side of the valve body (14) as one of the said prong-like protrusions (42).

3. A valve according to Claim 1 or Claim 2, further including coupling means (22, 36) for securing axially the valve body (14) to the housing (12) and limiting the rotation of the valve body (14) relative to the housing (12) between the said first and second positions.

4. A valve according to Claim 3, wherein the said coupling means (22, 36) include a horizontal groove (36) substantially extending through a 90-degree angle and a pin-like projection (22) axially locked in the groove (36) but able to slide along it.

5. A valve according to Claim 4, wherein the said horizontal groove (36) is provided in the valve body (14) and the said pin-like projection (22) is formed by the second tubular segment (18) of the housing (12).

6. A valve according to Claim 5, wherein the valve body (14) is coupled to the housing (12) by snap-fit of the pin-like projection (22) into the horizontal groove (36).

7. A valve according to any of the preceding claims, wherein the valve body (14) comprises a first, inner tubular segment (24) and a second, outer tubular segment (30) arranged coaxial with the first one and radially spaced therefrom so as to define a seat (32) for receiving the second tubular segment (18) of the housing (12).

8. A valve according to Claims 4 and 7, wherein the said horizontal groove (36) is provided in the outer tubular segment (30) of the valve body (14).

9. A valve according to Claim 7, wherein the valve body (14) further includes a pair of gripping arms (38) extending radially in opposite directions from the outer tubular segment (30).

10. A valve according to Claim 9, wherein the said gripping arms (38) of the valve body (14) extend parallel to the channel (44) so as to be aligned with the axis (X) of the first tubular segment (16) of the housing (12) when the valve is in the said first position.

## Patentansprüche

1. T-förmiges 2-Wegeventil (10) für eine Infusionsleitung, wobei das Ventil aufweist:
- ein Kupplungsgehäuse (12), das den Aufbau eines auf dem Kopf stehenden T besitzt, mit
einem ersten Rohrsegment (16), das entlang einer ersten Achse (X) verläuft und an seinen entgegengesetzten Enden einen ersten und einen zweiten Verbindungsteil (20) für eine Verbindung mit einer ersten Hauptleitung bildet, und
einem zweiten Rohrsegment (18), das entlang einer zweiten Achse (Z), die senkrecht zur ersten Achse (X) liegt, verläuft und sich in einen Mittelteil des ersten Segments (16) erstreckt;
- einen Ventilkörper (14), der im zweiten Segment (18) des Gehäuses (12) so befestigt ist, dass er um die zweite Achse (Z) zwischen einer ersten und einer zweiten Stellung gedreht werden kann; und
- einen dritten Anschlussteil (28) für eine Verbindung mit einer zweiten Infusionsleitung;
wobei der Ventilkörper (14) einen Endteil (42) besitzt, der im ersten Rohrsegment (16) des Gehäuses (12) aufgenommen wird und einen Kanal (44) bildet, der im Wesentlichen quer zur zweiten Achse (Z) so angeordnet ist, dass der Kanal (44) dann, wenn sich der Ventilkörper (14) in der ersten Stellung befindet, im Wesentlichen mit der ersten Achse (X) ausgerichtet ist, und der Kanal (44) dann, wenn sich der Ventilkörper (14) in der zweiten Stellung befindet, im Wesentlichen senkrecht zur ersten Achse (X) angeordnet ist; und
wobei der Ventilkörper (14) eine radiale Durchgangsöffnung (46) besitzt, die so ausgebildet ist, dass sie den dritten Verbindungsteil (28) dann mit einem stromabwärts liegenden Teil des ersten Segments (16) des Gehäuses (12) verbindet, wenn sich der Ventilkörper (14) in der zweiten Stellung befindet, wodurch die stromaufwärts liegende Seite der Hauptleitung geschlossen ist und die Fluidströmung von der Infusionsleitung zur stromabwärts liegenden Seite der Hauptleitung fließen kann;
wobei das Ventil **dadurch gekennzeichnet ist, dass** der Endteil (42) des Ventilkörpers (14) aus einem gabelförmigen Teil besteht, der ein Paar von zinkenartigen Vorsprüngen (42) besitzt, zwischen denen der Kanal (44) definiert ist, und dass die freien Enden der zinkenartigen Vorsprünge (42) des Ventilkörpers (14) in einen Sitz (43) eingreifen, der als Umfangsrille in einem Mittelteil des ersten Rohrsegments (16) des Gehäuses (12) ausgebildet ist, um eine Dichtheit gegenüber dem Fluid sicherzustellen, wenn sich das Ventil in der zweiten Stellung befindet.

2. Ventil gemäß Anspruch 1, wobei die radiale Durchgangsöffnung (46) auf derselben Seite des Ventilkörpers (14) wie einer der zinkenartigen Vorsprünge (42) angeordnet ist.

3. Ventil gemäß Anspruch 1 oder Anspruch 2, wobei das Ventil weiters eine Kupplungseinrichtung (22, 36) aufweist, um den Ventilkörper (14) des Gehäuses (12) axial zu befestigen und die Drehung des Ventilkörpers (14) relativ zum Gehäuse (12) zwischen der ersten und der zweiten Stellung zu begrenzen.

4. Ventil gemäß Anspruch 3, wobei die Kupplungseinrichtung (22, 36) eine horizontale Rille (36), die im Wesentlichen über einen Winkel von 90° verläuft, sowie einen zapfenartigen Vorsprung (22) aufweist, der in der Rille (36) axial verriegelt ist, aber in ihr gleiten kann.

5. Ventil gemäß Anspruch 4, wobei die horizontale Rille (36) im Ventilkörper (14) vorgesehen ist und der zapfenartige Vorsprung (22) vom zweiten Rohrsegment (18) des Gehäuses (12) gebildet wird.

6. Ventil gemäß Anspruch 5, wobei der Ventilkörper (14) mit dem Gehäuse (12) durch das Einschnappen des zapfenartigen Vorsprungs (22) in die horizontale Rille (36) gekuppelt wird.

7. Ventil gemäß irgendeinem der bisherigen Ansprüche, wobei der Ventilkörper (14) ein erstes inneres Rohrsegment (24) sowie ein zweites äußeres Rohrsegment (30) umfasst, das koaxial mit dem ersten angeordnet und radial von diesem beabstandet ist, um einen Sitz (32) für die Aufnahme des zweiten Rohrsegments (18) des Gehäuses (12) zu bilden.

8. Ventil gemäß Anspruch 4 und 7, wobei die horizontale Rille (36) im äußeren Rohrsegment (30) des Ventilkörpers (14) vorgesehen ist.

9. Ventil gemäß Anspruch 7, wobei der Ventilkörper (14) weiters ein Paar von Greifarmen (38) aufweist, die vom äußeren Rohrsegment (30) radial in entgegengesetzte Richtungen verlaufen.

10. Ventil gemäß Anspruch 9, wobei die Greifarme (38) des Ventilkörpers (14) so parallel zum Kanal (44) verlaufen, dass sie dann mit der Achse (X) des ersten Rohrsegments (16) des Gehäuses (12) ausgerichtet sind, wenn sich das Ventil in der ersten Stellung befindet.

## Revendications

1. Vanne à deux voies en forme de T (10) pour une ligne d'infusion, comprenant :
un boîtier de couplage (12) ayant une configuration en forme de T inversé, avec :
un premier segment tubulaire (16) s'étendant le long d'un premier axe (X) et formant au niveau de ses extrémités opposées des première et deuxième parties de raccordement (20) pour le raccordement avec une première ligne principale, et
un deuxième segment tubulaire (18) s'étendant le long d'un deuxième axe (Z) perpendiculaire au premier axe (X) et débouchant dans une partie centrale du premier segment (16) ;
un corps de vanne (14) monté de manière rotative dans le deuxième segment (18) du boîtier (12) autour du deuxième axe (Z) entre une première et une deuxième position ; et
une troisième partie de raccordement (28) pour le raccordement avec une deuxième ligne d'infusion ;
dans laquelle le corps de vanne (14) a une partie d'extrémité (42) qui est contenue à l'intérieur du premier segment tubulaire (16) du boîtier (12) et définit un canal (44) agencé sensiblement de manière transversale par rapport au deuxième axe (Z), de sorte que lorsque le corps de vanne (14) est dans ladite première position, le canal (44) est sensiblement aligné avec le premier axe (X) et lorsque le corps de vanne (14) est dans la deuxième position, le canal (44) est agencé de manière sensiblement perpendiculaire par rapport au premier axe (X) ; et
dans laquelle le corps de vanne (14) a un trou de passage radial (46) configuré pour raccorder la troisième partie de raccordement (28) avec une partie en aval du premier segment (16) du boîtier (12) lorsque le corps de vanne (14) est dans la deuxième position, moyennant quoi le côté en amont de la ligne principale est fermé et l'écoulement de fluide est autorisé à partir de la ligne d'infusion jusqu'au côté en aval de la ligne principale ;
la vanne étant **caractérisée en ce que** ladite partie d'extrémité (42) du corps de vanne (14) est une partie en forme de fourche ayant une paire de saillies en forme de dent (42) entre lesquelles le canal (44) est défini, et **en ce que** les extrémités libres des saillies en forme de dent (42) du corps de vanne (14) se mettent en prise dans un siège (43) formé comme une rainure circonférentielle dans une partie centrale du premier segment tubulaire (16) du boîtier (12) afin de garantir l'étanchéité au fluide lorsque la vanne est dans ladite deuxième position.

2. Vanne selon la revendication 1, dans laquelle le trou de passage radial (46) est agencé du même côté du corps de vanne (14) que celui desdites saillies en forme de dent (42).

3. Vanne selon la revendication 1 ou la revendication 2, comprenant en outre des moyens de soupape (22, 36) pour fixer axialement le corps de vanne (14) sur le boîtier (12) et limitant la rotation du corps de vanne (14) par rapport au boîtier (12) entre lesdites première et deuxième positions.

4. Vanne selon la revendication 3, dans laquelle lesdits moyens de couplage (22, 36) comprennent une rainure horizontale (36) s'étendant sensiblement sur un angle à 90 degrés et une saillie en forme de broche (22) axialement bloquée dans la rainure (36) mais pouvant coulisser le long de cette dernière.

5. Vanne selon la revendication 4, dans laquelle ladite rainure horizontale (36) est prévue dans le corps de vanne (14) et ladite saillie en forme de broche (22) est formée par le deuxième segment tubulaire (18) du boîtier (12).

6. Vanne selon la revendication 5, dans laquelle le corps de vanne (14) est couplé au boîtier (12) par ajustement avec serrage de la saillie en forme de broche (22) dans la rainure horizontale (36).

7. Vanne selon l'une quelconque des revendications précédentes, dans laquelle le corps de vanne (14) comprend un premier segment tubulaire interne (24) et un deuxième segment tubulaire externe (30) agencé de manière coaxiale avec le premier et radialement espacé de ce dernier afin de définir un siège (32) pour recevoir le deuxième segment tubulaire (18) du boîtier (12).

8. Vanne selon les revendications 4 et 7, dans laquelle ladite rainure horizontale (36) est prévue dans le segment tubulaire externe (30) du corps de vanne (14).

9. Vanne selon la revendication 7, dans laquelle le corps de vanne (14) comprend en outre une paire de bras de préhension (38) s'étendant de manière radiale dans des directions opposées du segment tubulaire externe (30).

10. Vanne selon la revendication 9, dans laquelle lesdits bras de préhension (38) du corps de vanne (14) s'étendent parallèlement au canal (44) afin d'être alignés avec l'axe (X) du premier segment tubulaire (16) du boîtier (12) lorsque la vanne est dans ladite première position.
